# FASCICULE DE BREVET EUROPEEN

(11) **EP 2 424 508 B1**
(45) Date de publication et mention de la délivrance du brevet: **11.03.2020**
(21) Numéro de dépôt: 10727061.3
(22) Date de dépôt: 28.04.2010
(51) Int. Cl.: A61K 9/20, A61K 9/48

(54) **COAGGLOMÉRATS DE MANNITOL ET D'AMIDON GRANULAIRE COMPRIMABLES ET À ÉCOULEMENT LIBRE**
KOMPRIMIERBARE UND FLIESSFÄHIGE KOAGGLOMERATE VON MANNITOL UND GRANULATFÖRMIGER STÄRKE
COMPRESSIBLE AND FREE-FLOW CO-AGGLOMERATES OF MANNITOL AND GRANULAR STARCH

(30) Priorité: 30.04.2009 FR 0952894
(43) Date de publication de la demande: 07.03.2012
(73) Titulaire: Roquette Frères, 62136 Lestrem (FR)
(72) Inventeur: BOIT, Baptiste, 62400 BETHUNE (FR); FRANCOIS, Alain, 62232 Hinges (FR); LEFEVRE, Philippe, 59660 Haverskerque (FR); PASSE, Damien, 59500 Douai (FR)
(74) Mandataire: Plasseraud IP
(86) Numéro de dépôt international: PCT/FR2010/050813
(87) Numéro de publication internationale: WO 2010/125313

(56) Documents cités:
- EP-A- 1 153 616
- WO-A1-2010/001063
- US-A1- 2004 180 085
- DATABASE CA [Online] CHEMICAL ABSTRACTS SERVICE, COLUMBUS, OHIO, US; 22 mai 1997 (1997-05-22), ASOGAWA, TATSUO ET AL: "Vitamin B granules" XP002560481 extrait de STN Database accession no. 1997:326257 -& JP 9 077669 A (TAKEDA CHEMICAL INDUSTRIES, LTD., JAPAN) 25 mars 1997 (1997-03-25) -& DATABASE WPI Week 199722 1997 Thomson Scientific, London, GB; AN 1997-241649 XP002560482 "Vitamin B granules" & JP 9 077669 A (TAKEDA CHEM IND LTD) 25 mars 1997 (1997-03-25)
- DATABASE WPI Week 198624 1986 Thomson Scientific, London, GB; AN 1986-152485 XP002560483 & JP 61 085331 A (FUJI CHEM IND CO LTD) 30 avril 1986 (1986-04-30)

## Description

La présente invention a pour objet des coagglomérats de mannitol cristallin et d'amidon granulaire qui présentent une comprimabilité remarquablement élevée, ce qui les destine tout particulièrement à la préparation de comprimés.

L'invention est également relative à des coagglomérats de mannitol cristallin et d'amidon granulaire présentant une excellente aptitude à l'écoulement, ce qui les destine également à être utilisés pour le remplissage de gélules.

L'invention est enfin relative au procédé permettant d'obtenir ces coagglomérats.

Dans le domaine de l'utilisation des polyols, en ce qui concerne le domaine auquel on s'intéressera spécifiquement dans la présente invention, à savoir les excipients pharmaceutiques et les édulcorants massiques utilisés dans l'industrie alimentaire, plusieurs polyols pulvérulents sont d'usage courant. Il s'agit du sorbitol, du xylitol et surtout du mannitol.

L'industrie pharmaceutique est consommatrice de tonnages importants de mannitol. Celui-ci est notamment utilisé en tant qu'excipient dans les formes sèches que sont par exemple les poudres de remplissage des gélules, les poudres pour sachet à disperser ou à dissoudre extemporanément dans l'eau, les formes solides orales et les comprimés.

En effet le mannitol, en raison de sa saveur et de la très faible hygroscopicité de sa forme cristalline, constitue un excellent excipient, notamment pour sa très grande inertie chimique vis-à-vis des principes actifs.

Malheureusement, le produit obtenu par cristallisation dans l'eau à partir d'une solution sursaturée, n'est pas naturellement compressible.

Pour remédier à cette situation, il est connu par les spécialistes du domaine d'ajouter un liant pour augmenter sa comprimabilité.

Le brevet US 3,145,146 décrit par exemple un procédé consistant à utiliser de la paraffine, de la gomme ou un dérivé de la cellulose comme liant avant l'étape d'atomisation conduisant à la préparation de la poudre de mannitol.

Cette solution technique n'est cependant pas prisée par l'utilisateur.

Pour la préparation des gélules, il est connu de l'homme du métier que les mélanges susceptibles de servir au remplissage des gélules doivent pouvoir se prêter au remplissage automatique, afin de garantir un dosage uniforme.

Il va de soi qu'un travail de formulation galénique est nécessaire pour certaines substances, afin d'assurer un remplissage uniforme et précis sur machines à très grande vitesse.

Il convient en particulier de veiller aux paramètres suivants :
- forme et taille des particules,
- granulométrie uniforme,
- homogénéité du mélange,
- propriété d'écoulement de la poudre,
- taux d'humidité,
- bonne agglomération sous pression.

Or, à côté de sa non comprimabilité, il faut ajouter un autre inconvénient du mannitol obtenu par cristallisation dans l'eau qui est qu'il présente des propriétés d'écoulement médiocres en raison de la structure orthorhombique de ses cristaux, et surtout en raison de sa friabilité excessive.

Cette friabilité conduit à la formation de fines particules qui nuisent particulièrement à ses propriétés d'écoulement, en particulier lors du remplissage et de la vidange des trémies et des goulottes d'alimentation des dispositifs mis en œuvre pour fabriquer des comprimés, ou pour remplir des gélules.

De ce fait, le mannitol cristallisé dans l'eau n'est absolument pas un excipient de choix utilisable pour le remplissage de gélules.

Ici encore, il est nécessaire d'ajouter un agent liant.

Dans le brevet US 3,145,146, l'ajout d'agent liant n'empêche cependant pas qu'au moins 50 % des particules de la poudre ont encore une taille inférieure à 75 µm, ce qui est loin d'être idéal pour obtenir un bon écoulement.

Dans le domaine des comprimés, l'amidon peut être avantageusement utilisé comme liant lorsqu'on lui fait subir une cuisson préalable, et peut également servir de diluant.

Il possède par ailleurs de bonnes propriétés désintégrantes du fait de son hydrophilie dans l'eau lorsqu'il est employé à l'état de granule.

Il doit cependant être incorporé en quantité élevée, généralement supérieure à 15 % de la formulation finale.

Par contre, il présente du fait de la faible taille de ses particules et de sa faible masse volumique, l'inconvénient de ne pas s'écouler.

L'élasticité importante de ses granules lui confère par ailleurs une piètre comprimabilité qui ne permet pas la fabrication de comprimés de dureté satisfaisante.

Il ne peut donc être envisagé par l'homme du métier d'associer le mannitol et l'amidon granulaire afin de mettre à profit les propriétés liantes de l'amidon pour apporter au mannitol les propriétés de comprimabilité ou d'écoulement qui lui font défaut.

En fait, si l'association de l'amidon et du mannitol a été travaillée, c'est plutôt dans le domaine de la fabrication des comprimés orodispersibles, où il s'est surtout agi de mettre à profit les propriétés désintégrantes et diluantes de l'amidon.

Par exemple dans la demande de brevet WO 00/47233, il est décrit une préparation pour comprimés comprenant le mélange physique d'un principe actif, un amidon, avec notamment du mannitol. Or, il est fortement recommandé dans cette demande de brevet d'ajouter à ce mélange physique un ou plusieurs lubrifiants choisis parmi le stéarate de magnésium, le stéarate de calcium, le stéarylfumarate de sodium et l'anhydride silicique léger pour faciliter l'écoulement desdits mélanges dans les équipements de fabrication de comprimés.

Dans la demande de brevet JP 09.077669, il est décrit un mélange physique contenant du mannitol, de l'amidon et de la vitamine C. Ledit mélange physique est séché par un procédé d'atomisation classique, c'est-à-dire une atomisation de type simple effet sans recyclage des fines. Il en résulte une poudre peu comprimable et présentant un mauvais écoulement.

Il apparaît ainsi difficile de proposer une solution unique permettant de concilier à la fois les propriétés de comprimabilité et l'aptitude à l'écoulement d'une poudre de mannitol cristallin, et c'est aller à l'encontre d'un préjugé technique que de proposer une solution consistant à utiliser un agglomérat de mannitol et d'amidon granulaire.

C'est ainsi que même la société Demanderesse dans son brevet EP 1.138.661, réussissait à conférer à un mannitol cristallin de fine granulométrie une excellente aptitude à l'écoulement, mais échouait à lui attribuer des propriétés de comprimabilité.

Le mannitol pulvérulent obtenu selon ledit brevet EP 1.138.661 avait ainsi été exemplifié dans la formulation d'une poudre de remplissage de gélules, mais le remplissage avait dû être simulé sur un appareil spécialement conçu pour l'expérimentation.

L'invention a donc pour but de remédier à ces inconvénients et la société Demanderesse a eu le mérite de trouver, après de nombreux travaux, que ce but pouvait être atteint dès lors que l'on utilise des coagglomérats à base d'amidon granulaire et de mannitol tels que définis dans la revendication 1.

Par amidon granulaire, on entend les amidons natifs de toutes origines, naturels ou hybrides, de type granulaire, et tous les amidons modifiés chimiquement ayant gardé une forme granulaire.

On utilisera de préférence un amidon de maïs blanc tel que celui commercialisé par la société Demanderesse sous l'appellation « amidon extra-blanc » qui permet d'obtenir des granules d'une blancheur tout à fait satisfaisante.

L'invention a également pour objet des coagglomérats de mannitol cristallin et d'amidon granulaire caractérisés en ce qu'ils permettent :
- la préparation par compression directe, de comprimés présentant de remarquables propriétés de dureté,
- le remplissage de gélules pour la préparation desquelles il n'est pas nécessaire d'utiliser de grandes quantités de lubrifiant et d'agent d'écoulement pour faciliter le remplissage et la vidange des trémies et des goulottes d'alimentation des dispositifs mis en œuvre.

L'invention a ainsi pour objet l'utilisation des coagglomérats de mannitol cristallin et d'amidon granulaire où l'amidon granulaire participe à la comprimabilité dudit agglomérat et facilite la lubrification, autant de propriétés de l'amidon granulaire que l'homme du métier ne s'attend pas à trouver.

L'invention a enfin pour objet :
- des comprimés
- des gélules
contenant des coagglomérats de mannitol cristallin et d'amidon granulaire où l'amidon granulaire agit à la fois comme agent liant et comme agent lubrifiant.

L'invention a pour objet des coagglomérats de mannitol cristallin et d'amidon granulaire caractérisés en ce qu'ils présentent :
- une comprimabilité, déterminée selon un test A supérieure à 120 N, de préférence comprise entre 200 et 450 N, et plus préférentiellement encore comprise entre 220 et 400N,
- une note d'écoulement, déterminée selon le test B, comprise entre 3 et 15 secondes, de préférence comprise entre 4 et 8 secondes et plus préférentiellement encore comprise entre 4 et 6 secondes et
- un ratio mannitol / amidon compris entre 99,5/0,5 et 50/50, et de préférence entre 95/5 et 70/30.

Les coagglomérats selon l'invention sont caractérisés par leur comportement en compression, déterminé selon un test A.

Le test A consiste à mesurer la force, exprimée en Newtons, qui est nécessaire pour provoquer l'écrasement d'un comprimé préparé à l'aide d'une presse alternative de laboratoire FROGERAIS AM commercialisée par la société SVIAC (FRANCE) à partir dudit coagglomérat lubrifié avec 1,2 % de stéarate de magnésium (lubrification réalisée par le mélange pendant 5 minutes du coagglomérat et du stéarate de magnésium dans un mélangeur TURBULA T2C de WILLY A. BACHOFEN), traduisant donc la résistance à l'écrasement du comprimé qui est cylindrique à faces convexes d'un diamètre de 13 mm, d'une épaisseur de 6 mm et d'un poids de 0,734 g, i.e. d'une masse volumique apparente de 1,3 g/ml. Cette force, exprimant la dureté du comprimé et par conséquent la comprimabilité de la poudre, est mesurée sur un duromètre ERWEKA TBH 30 GMD. La valeur donnée en Newtons correspond à une moyenne réalisée sur 10 mesures.

Les coagglomérats selon l'invention présentent alors une comprimabilité, déterminée selon un test A supérieure à 120 N, de préférence comprise entre 200 et 450 N.

L'amidon possédant une piètre comprimabilité, il est surprenant de constater que les dits coagglomérats de mannitol et d'amidon présentent une comprimabilité élevée. Ce qui permet de réaliser des comprimés qui bien que contenant en plus des actifs pharmaceutiques, vétérinaires ou nutraceutiques, ou des produits alimentaires ou des excipients, arômes, colorants, conserveront une dureté suffisante pour leur manipulation ultérieure, que ce soit pelliculage, conditionnement en blister, en tube, voire en vrac.

Une comprimabilité importante est aussi recherchée pour le remplissage des gélules, notamment sur les géluleuses industrielles utilisant les principes du compresso-doseur ou du disque doseur.

Les coagglomérats selon l'invention sont également caractérisés par leur aptitude à l'écoulement, déterminée selon un test B.

Le test B consiste à déterminer le temps nécessaire à l'écoulement de 100 g de poudre selon la méthode de mesure préconisée par la Pharmacopée Européenne (PE 5.0 tome 1, 01/2005: 20916, paragraphe 2.9.1.6 ; équipement selon la figure 2.9.16.-2).

Les coagglomérats selon l'invention présentent alors une note d'écoulement, déterminée selon le test B, comprise entre 3 et 15 secondes, de préférence comprise entre 4 et 8 secondes.

Les coagglomérats présentent un excellent écoulement libre alors que l'amidon granulaire ne s'écoule pas librement et que les mélanges physiques de mannitol et d'amidon ne s'écoulent pas ou très peu.

Les coagglomérats selon l'invention présentent un diamètre moyen volumique laser D4,3 compris entre 60 et 500 µm, de préférence compris entre 100 et 250 µm.

Les valeurs de répartition granulométrique sont déterminées sur un granulomètre à diffraction LASER type LS 200 de la société BECKMAN-COULTER, équipé de son module de dispersion poudre (voie sèche), en suivant le manuel technique et les spécifications du constructeur.

Les conditions opératoires de vitesse de vis sous trémie et d'intensité de vibration de la goulotte de dispersion sont déterminées de manière à ce que la concentration optique soit comprise entre 4 % et 12 %, idéalement de 8 %.

La gamme de mesure du granulomètre à diffraction LASER type LS 200 est de 0,04 µm à 2.000 µm. Les résultats sont calculés en % volumique, et exprimés en µm.

La courbe de distribution granulométrique permet également de déterminer la valeur du diamètre moyen volumique (moyenne arithmétique) D4,3.

Dans les comprimés et les gélules contenant des actifs, l'homogénéité du dosage en actifs est obtenue en optimisant le diamètre moyen des excipients par rapport au diamètre moyen des actifs, généralement, sans que ce soit systématique, en rapprochant ces diamètres moyens.

Les coagglomérats selon l'invention donnent accès à des diamètres moyens correspondant à la majorité des actifs, de 60 à 500 µm, autant pour la réalisation de comprimés que pour le remplissage de gélules.

Les coagglomérats selon l'invention sont également caractérisés en ce que le ratio mannitol / amidon est compris entre 99,5/0,5 et 50/50, et de préférence entre 95/5 et 70/30.

Au-delà de 99,5 % de mannitol ou en deçà de 50 % de mannitol, la société Demanderesse a constaté que lesdits coagglomérats ne possédaient pas de propriétés de comprimabilité ou d'écoulement satisfaisantes.

De préférence, on choisit un ratio de mannitol et d'amidon compris entre 95/5 et 70/30.

Selon un autre mode de réalisation de l'invention, les coagglomérats comprennent du mannitol et de l'amidon et peuvent de plus contenir tout additif approprié dès lors qu'il ne nuise pas aux propriétés recherchées des granules finales, comme notamment des arômes, des colorants, des agents stabilisants, des liants, des lubrifiants, des conservateurs.

Il peut s'agir également de principes actifs pharmaceutiques ou phytosanitaires, de détergents.

Les coagglomérats conformes à l'invention présentent :
- une masse volumique aérée comprise entre 0,400 et 0,750 g/ml, de préférence comprise entre 0,450 et 0,650 g/ml, et
- une masse volumique tassée comprise entre 0,500 et 0,850 g/ml, de préférence comprise entre 0,550 et 0,750 g/ml.

Les masses volumiques aérées et tassées des coagglomérats conformes à l'invention sont les masses volumiques apparentes aérées et tassées déterminées selon la méthode de mesure préconisée par la Pharmacopée Européenne (PE 5.1 tome 1, 01/2005 : 20915 paragraphe 2-9-15 ; équipement selon la figure 2-9-15-1), la masse volumique apparente tassée étant obtenue après 1.250 tassements.

Pour diminuer le volume des comprimés et des gélules à avaler, et donc améliorer l'observance médicamenteuse des patients, des masses volumiques tassées élevées sont recherchées.

Néanmoins, une homogénéité de mélange entre le coagglomérat et la poudre d'actifs nécessite souvent une similitude dans les masses volumiques aérées. Il faut donc que le coagglomérat soit plus ou moins dense en fonction de l'actif à incorporer.

Les coagglomérats selon l'invention peuvent présenter des masses volumiques sur une plage importante s'adaptant à la majorité des cas.

Les coagglomérats de mannitol cristallin et d'amidon granulaire selon l'invention sont enfin caractérisés en ce que le mannitol se présente à la fois sous les formes cristallines alpha et bêta.

Le mannitol est classiquement commercialisé sous trois formes cristallines : alpha, bêta et delta, la forme bêta étant la forme la plus communément utilisée car d'une plus grande stabilité.

Il est en effet connu en toute généralité que le mannitol de forme cristalline alpha ou delta, mis en présence d'une faible quantité d'eau était instable, car se dissolvait puis recristallisait sous forme bêta.

La société Demanderesse a donc été à l'encontre d'un préjugé technique qui considère qu'il faut préférer la forme cristalline bêta à la forme alpha, notamment dans des applications où le risque d'une recristallisation de la forme alpha en forme bêta est néfaste, par exemple pour l'aptitude à la désintégration en bouche de comprimés en contenant, ou pour tenter d'optimiser les propriétés de comprimabilité, ou l'aptitude à l'écoulement, du mannitol.

La détermination de la coexistence des deux formes cristallines alpha et bêta du mannitol peut être réalisée classiquement par toute méthode connue par ailleurs de l'homme du métier, i.e. par spectrométrie infrarouge ou par diffraction X.

Par spectrométrie infrarouge, la méthode communément utilisée est dite du pastillage au bromure de potassium. Les deux formes cristallines du mannitol présentent alors un profil différent.

Localement, les différences sont plus ou moins marquées ; la zone spectrale étudiée est ainsi localisée entre 2850 et 3050 cm⁻¹ ; la forme alpha se caractérise par la présence d'une bande d'adsorption vers 2885 cm⁻¹, la forme bêta se caractérise quant à elle par celles vers 2985 et 2900 cm⁻¹.

Les mélanges de ces deux formes montreront alors des différences plus ou moins prononcées selon les proportions des deux formes et la zone spectrale étudiée.

Grâce à des mesures effectuées sur des mélanges étalons de formes cristallines alpha et bêta, il est relativement aisé de quantifier les proportions de formes cristallines dans les coagglomérats de mannitol cristallin et d'amidon granulaire selon l'invention, comme il sera exemplifié ci-après.

Une confirmation de ces valeurs peut être réalisée par spectrométrie de diffraction des rayons X.

L'homme du métier sait en effet que la quantification relative est rendue possible au moyen du rapport d'intensité des raies de diffraction : la hauteur de la raie à 27°2 correspondant à la forme cristalline bêta par rapport à la hauteur de la raie à 20°1 correspondant à la forme alpha.

Un mélange présentant par exemple 25 % de forme cristalline alpha et 75 % de forme cristalline bêta donne un rapport de hauteur des raies de 21, alors qu'un mélange à 50 % donne une valeur de rapports de raies de 5.

Les coagglomérats conformes à l'invention possédant les caractéristiques mentionnées ci-dessus sont susceptibles d'être obtenus tout particulièrement selon un procédé qui comprend une étape d'atomisation ou de granulation d'un mélange de mannitol et d'amidon.

Ce but n'avait pas été atteint jusqu'alors au moyen des procédés connus de l'homme du métier, et applicables à la fois au mannitol et à l'amidon. Ce dernier présente en effet l'inconvénient, lorsque l'on utilise des procédés thermiques en milieu aqueux, de cuire et de perdre ainsi son caractère granulaire.

Dans un premier mode préférentiel de réalisation du procédé conforme à l'invention, les coagglomérats de mannitol cristallin et d'amidon sont préparés par atomisation ou par granulation d'un mélange d'une solution de mannitol et d'amidon, l'amidon étant mis en suspension dans la solution de mannitol.

Dans un deuxième mode préférentiel de réalisation du procédé conforme à l'invention, les coagglomérats de mannitol cristallin et d'amidon sont préparés par atomisation ou par granulation d'un mélange d'une solution de mannitol et d'amidon sec, l'amidon étant incorporé sous forme sèche dans la tour d'atomisation ou lors de l'étape de granulation.

Selon un premier mode préférentiel, on peut procéder selon les étapes suivantes :
a) préparer à une température comprise entre 45 et 65°C une « solution » de mannitol et d'amidon granulaire dans laquelle :
   - le ratio mannitol/amidon est compris entre 99,5/0,5 et 50/50, et de préférence entre 95/5 et 70/30
   - la matière sèche est comprise entre 25 et 45 % en poids sec,
b) maintenir ladite solution de mannitol et d'amidon à une température comprise entre 45 et 65°C,
c) atomiser ladite solution dans une tour d'atomisation de type MSD équipée d'une buse d'atomisation haute pression avec recyclage des fines particules en tête de tour,
d) récupérer les coagglomérats de mannitol et d'amidon ainsi obtenus.

La première étape consiste donc à préparer à une température comprise entre 45 et 65°C une solution de mannitol et d'amidon granulaire dans laquelle :
- le ratio mannitol/amidon est compris entre 99,5/0,5 et 50/50, et de préférence entre 95/5 et 70/30,
- la matière sèche est comprise entre 25 et 45 % en poids sec.

Si la granulométrie initiale des cristaux de mannitol n'est pas une caractéristique essentielle, la teneur en amidon est au contraire un paramètre important, ainsi que son rapport au mannitol.

Il est alors choisi un ratio mannitol/amidon compris entre 99,5/0,5 et 50/50, et de préférence entre 95/5 et 70/30.

La deuxième étape consiste à maintenir ladite solution de cristaux de mannitol et d'amidon à la température comprise entre 45 et 65°C.

Le maintien à cette température permet de conserver le mannitol à l'état dissous ou sous formes de cristaux de faible granulométrie. La température est choisie de façon à conserver l'amidon sous forme granulaire.

La troisième étape consiste alors à atomiser ladite solution dans une tour d'atomisation de type MSD (i.e. Multi Stage Dryer) équipée d'une buse d'atomisation haute pression avec recyclage des fines particules en tête de tour.

Comme il sera exemplifié ci-après la société Demanderesse recommande d'utiliser une tour de type MSD 20 commercialisée par la société NIRO.

La buse de pulvérisation est choisie de manière à obtenir une pression comprise entre 20 et 250 bars, pour un débit compris entre 50 et 170 l/h, de préférence de l'ordre de 120 l/h.

La température des airs d'entrée sont réglées de la manière suivante :
- pour l'air d'entrée en amont de la tête de tour : température comprise entre 120°C et 240°C,
- pour le lit fluidisé statique : température comprise entre 50 et 120°C,
- pour le lit fluidisé vibré : température de l'ordre de 20°C.

La température de sortie de tour est alors comprise entre 50 et 120°C.

Les coagglomérats selon l'invention sont enfin récupérés en sortie de tour d'atomisation.

Selon un second mode préférentiel, on peut procéder selon les étapes suivantes :
a) préparer à une température comprise entre 45 et 90°C une solution de mannitol dans laquelle la matière sèche est comprise entre 25 et 50 % en poids sec,
b) maintenir ladite solution de mannitol à une température comprise entre 45 et 90°C,
c) atomiser ladite solution dans une tour d'atomisation de type MSD équipée d'une buse d'atomisation haute pression avec recyclage des fines particules en tête de tour, en injectant via un doseur pondéral l'amidon sec dans le système de recyclage des fines dans un ratio mannitol/amidon compris entre 99,5/0,5 et 50/50, et de préférence entre 95/5 et 70/30
d) récupérer les coagglomérats de mannitol et d'amidon ainsi obtenus.

La société Demanderesse a ainsi observé que l'injection de l'amidon dans le système de recyclage permet un mélange intime avec le mannitol lors du séchage.

La solution est atomisée, comme dans le mode préférentiel précédent, dans une tour d'atomisation de type MSD (i.e. Multi Stage Dryer) équipée d'une buse d'atomisation haute pression avec recyclage des fines particules en tête de tour.

Comme il sera exemplifié ci-après la société Demanderesse recommande d'utiliser une tour de type MSD 20 commercialisée par la société NIRO.

La buse de pulvérisation est choisie de manière à obtenir une pression comprise entre 20 et 250 bars, pour un débit compris entre 50 et 170 l/h, de préférence de l'ordre de 120 l/h.

Les températures des airs d'entrée sont réglées de la manière suivante :
- pour l'air d'entrée en amont de la tête de tour : température comprise entre 120°C et 240°C,
- pour le lit fluidisé statique : température comprise entre 50 et 120°C,
- pour le lit fluidisé vibré : température de l'ordre de 20°C.

La température de sortie de tour est alors comprise entre 50 et 120°C.

Les coagglomérats selon l'invention sont enfin récupérés en sortie de tour d'atomisation.

Selon un troisième mode préférentiel, on peut procéder selon les étapes suivantes :
a) préparer à une température comprise entre 45 et 65°C une solution de mannitol et d'amidon granulaire dans laquelle :
   - le ratio mannitol/amidon est compris entre 99,5/0,5 et 50/50, et de préférence entre 95/5 et 70/30
   - la matière sèche est comprise entre 25 et 45 % en poids sec,
b) maintenir ladite solution de mannitol et d'amidon à une température comprise entre 45 et 65°C,
c) granuler par pulvérisation de ladite solution dans un granulateur à lit d'air fluidisé,
d) récupérer les coagglomérats de mannitol et d'amidon ainsi obtenus.

Pour procéder à la granulation, on peut employer par exemple un granulateur à lit d'air fluidisé continu.

Il peut être choisi avantageusement un granulateur à lit d'air fluidisé continu circulaire avec tube de déchargement, ou continu rectangulaire à écoulement piston.

Comme il sera exemplifié ci-après, la société Demanderesse a choisi d'employer un granulateur à lit d'air fluidisé continu avec classificateur de type AGT commercialisé par la société GLATT.

L'invention sera mieux comprise à l'aide des exemples qui suivent, lesquels se veulent illustratifs et non limitatifs.

### Exemple 1 : Préparation de coagglomérats selon l'invention par atomisation du mélange d'une solution de mannitol dans laquelle l'amidon granulaire est mis en suspension

On prépare par atomisation selon l'invention différentes compositions de coagglomérats consistant en mannitol et en amidon granulaire à des ratios respectivement de 99/1, 95/5, 90/10, 85/15 et 80/20.

On utilise du mannitol cristallisé, commercialisé par la Société Demanderesse sous le nom PEARLITOL® 50C présentant un diamètre moyen volumique laser d'environ 50 µm et de l'amidon de maïs « extra-blanc ».

On prépare une solution de mannitol et d'amidon à la matière sèche désirée en dissolvant le mannitol cristallisé dans de l'eau déminéralisée à 55°C, et en y mettant en suspension l'amidon extra blanc de maïs.

L'agitation doit permettre d'obtenir une solution fluide, homogène et sans grumeaux. Les conditions opératoires de fabrication de ces coagglomérats dans la tour d'atomisation de type MSD 20 commercialisée par la société NIRO sont figurées dans le tableau 1 suivant.

**Tableau 1**

| Coagglomérats Selon l'invention | Rapport mannitol / amidon | Matière sèche totale (%) | Pression (bars) | Buse (SPRAYING SYSTEM type SK) | Tp air amont (°C) | Tp du lit fluidisé statique (°C) | Tp air de sortie (°C) |
|---|---|---|---|---|---|---|---|
| « A » | 99/1 | 30 | 42 | 60*21 | 175 | 77 | 66 |
| « B » | 95/5 | 31,6 | 70 | 60*21 | 200 | 80 | 72 |
| « C » | 90/10 | 32 | 50 | 60*21 | 170 | 70 | 50 |
| « D » | 85/15 | 33,6 | 50 | 60*21 | 228 | 80 | 89 |
| « E » | 80/20 | 32 | 50 | 56*21 | 200 | 80 | 63 |
| Témoin mannitol seul EP 1.138.661 | 100/0 | NS | NS | NS | NS | NS | NS |

Lors de la modification du taux d'amidon, la préparation des coagglomérats conformes à l'invention nécessite plus particulièrement des ajustements dans le choix de la buse, de la pression d'atomisation et des températures d'air pour obtenir une granulométrie moyenne similaire.

Les caractéristiques des coagglomérats de mannitol et d'amidon selon l'invention sont présentées dans le tableau 2 suivant.

**Tableau 2**

| Coagglomérats selon l'invention | Formes cristallines Alpha / Bêta (%) | Comprimabilité (N) | Note d'écoulement (s) | Granulométrie laser (D4,3 - µm) | Masse volumique tassée (g/ml) | Masse volumique aérée (g/ml) |
|---|---|---|---|---|---|---|
| « A » | 80/20 | 279 | 4 - 5 | 165 | 0,633 | 0,543 |
| « B » | 85/15 | 231 | 3 - 4 | 119 | 0,719 | 0,621 |
| « C » | 75/25 | 377 | 4 - 5 | 124 | 0,565 | 0,481 |
| « D » | 70/30 | 184 | 4 - 5 | 127 | 0,654 | 0,559 |
| « E » | 70/30 | 205 | 5 - 6 | 120 | 0,667 | 0,562 |
| Témoin mannitol seul EP 1.138.661 | Bêta | 60 à 80 N | 5 - 7 | 101 à 126 | 0,70 - 0,71 | 0,53 - 0,54 |

Le comportement des coagglomérats de mannitol selon l'invention est tout à fait satisfaisant en termes de comprimabilité et d'écoulement.

Avec des teneurs en amidon faible (1%) ou plus élevée (20%), le coagglomérat selon l'invention présente une comprimabilité élevée.

Cette comprimabilité élevée permet de formuler des comprimés qui conservent une dureté et donc une cohésion suffisante après incorporation d'une teneur élevée en principe actif.

C'est donc une caractéristique essentielle et recherchée pour un excipient pharmaceutique dédié aux comprimés et gélules. De plus, les temps d'écoulement mesurés sont très courts ce qui permettra une utilisation industrielle à très haute cadence sur des presses à comprimer et des géluleuses.

### Exemple 2 : Préparation de coagglomérats selon l'invention par atomisation d'une solution de mannitol et incorporation d'amidon granulaire sous forme sèche dans la tour d'atomisation.

On prépare par atomisation selon l'invention différentes compositions de coagglomérats consistant en mannitol et en amidon à des ratios respectivement de 80/20, 70/30 et 50/50.

On utilise du mannitol cristallisé, commercialisé par la société Demanderesse sous le nom PEARLITOL® 50C présentant un diamètre moyen volumique laser d'environ 50 µm et de l'amidon de maïs « extra-blanc ».

On prépare une solution de mannitol à la matière sèche désirée en dissolvant le mannitol cristallisé dans de l'eau déminéralisée à 70 °C.

L'amidon est introduit sous forme sèche dans le recyclage des fines via un doseur pondéral commercialisé par la société K-TRON.

Les conditions opératoires de fabrication de ces coagglomérats dans la tour d'atomisation de type MSD 20 commercialisée par la société NIRO sont figurées dans le tableau 3 suivant.

**Tableau 3**

| Coagglomérats Selon l'invention | Rapport mannitol /amidon | Matière sèche (%) | Pression (bars) | Buse (SPRAYING SYSTEM type SK) | Tp air amont (°C) | Tp du lit fluidisé statique (°C) | Tp air de sortie (°C) |
|---|---|---|---|---|---|---|---|
| « F » | 80/20 | 40 | 25 | 56*21 | 156 | 84 | 64 |
| « G » | 80/20 | 40 | 110 | 69*21 | 150 | 84 | 65 |
| « H » | 80/20 | 40 | 40 | 60*21 | 133 | 70 | 58 |
| « I » | 70/30 | 40 | 40 | 60*21 | 133 | 77 | 60 |
| « J » | 50/50 | 40 | 40 | 60*21 | 133 | 77 | 60 |

Les conditions de préparation des coagglomérats H, I, J selon l'invention montrent qu'aux mêmes paramètres, l'augmentation du taux d'amidon fait diminuer la granulométrie moyenne.

Celles relatives à la préparation des coagglomérats F et G montrent que plus la pression d'atomisation est importante et plus la granulométrie moyenne diminue.

Les caractéristiques des coagglomérats de mannitol et d'amidon selon l'invention sont présentées dans le tableau 4 suivant, en comparaison avec deux mélanges physiques P1 et P2 :
- le mélange P1 étant constitué de 80 % de PEARLITOL® 50 C et 20 % d'amidon « extra-blanc »,
- le mélange P2 étant constitué de 80 % de PEARLITOL® 200 SD (commercialisé par la société Demanderesse) et 20 % d'amidon « extra-blanc ».

**Tableau 4**

| Coagglomérats selon l'invention | Formes cristallines Alpha / Bêta (%) | Comprimabilité (N) | Note d'écoulement (s) | Granulométrie laser (D4,3 - µm) | Masse volumique tassée (g/ml) | Masse volumique aérée (g/ml) |
|---|---|---|---|---|---|---|
| « F » | 75/25 | 229 | 7 - 8 | 178 | 0,565 | 0,472 |
| « G » | 90/10 | 262 | 5 - 6 | 98 | 0,676 | 0,575 |
| « H » | 85/15 | 312 | 5 - 6 | 173 | 0,568 | 0,483 |
| « I » | 90/10 | 262 | 5 | 145 | 0,595 | 0,505 |
| « J » | 95/5 | 220 | 5 | 131 | 0,599 | 0,508 |
| Mélange de poudres P1 | 0/100 | Impossible | 5 à 6 | 34 | 0,667 | 0,548 |
| Mélange de poudres P2 | 50/50 | Impossible | Infini | 148 | 0,769 | 0,538 |

Le comportement des coagglomérats de mannitol selon l'invention est tout à fait satisfaisant en termes d'écoulement et de comprimabilité.

Il est possible de faire varier les paramètres de production pour ajuster la granulométrie et la masse volumique de la poudre.

L'homogénéité de mélange d'un excipient et d'un actif dépend du procédé de mélange mis en œuvre mais aussi de la similitude des caractéristiques des deux poudres.

Il est plus facile de mélanger une poudre de principe actif et une poudre d'excipient quand ils présentent la même granulométrie et la même masse volumique.

D'où l'intérêt de faire varier ces caractéristiques et de disposer d'une gamme de poudres de propriétés différentes pour s'adapter aux différentes poudres de principes actifs. De plus, il est possible de faire varier la teneur en amidon du coagglomérat pour ajuster la comprimabilité au produit à formuler sans perturber les remarquables propriétés d'écoulement de la poudre.

Un simple mélange physique de poudres (P1 et P2) ne permet pas d'obtenir les comprimés recherchés.

### Exemple 3 : Préparation de coagglomérats selon l'invention par granulation du mélange d'une solution de mannitol et d'amidon, l'amidon étant mis en suspension dans la solution de mannitol

On prépare par granulation selon l'invention différentes compositions de coagglomérats consistant en mannitol et en amidon à des ratios respectivement de 85/15, 70/30 et 50/50.

On utilise du mannitol cristallisé, commercialisé par la société Demanderesse sous le nom PEARLITOL® 50C présentant un diamètre moyen volumique laser d'environ 50 µm et de l'amidon de maïs « extra-blanc ».

On prépare une solution de mannitol et d'amidon à la matière sèche désirée en dissolvant le mannitol cristallisé dans de l'eau déminéralisée à 55°C et en mettant en suspension l'amidon extra blanc de maïs.

L'agitation doit permettre d'obtenir une solution fluide, homogène et sans grumeaux.

Les conditions opératoires de fabrication de ces coagglomérats dans le granulateur à lit d'air fluidisé continu de type AGT 150 commercialisé par la société GLATT sont figurées dans le tableau 5 suivant.

La buse de pulvérisation est en position « bottom spray ».

**Tableau 5**

| Coagglomérats Selon l'invention | Rapport mannitol / amidon | Matière sèche totale (%) | Débit alimentation (g/h) | Débit d'air (m3/h) | Tp air entrée (°C) | Tp lit produit (°C) | Pression pulvérisation buse (bars) | Pression classificateur zig-zag (bars) |
|---|---|---|---|---|---|---|---|---|
| « K » | 70/30 | 38 | 2700 | 105 | 105 | 55 | 1,0 | 0,25 |
| « L » | 50/50 | 45 | 2140 | 85 | 100 | 58 | 1, 0 | 0,3 |
| « M » | 85/15 | 34 | 4000 | 100 | 125 | 50 | 1,5 | 0,25 |
| « N » | 85/15 | 39 | 3700 | 100 | 125 | 62 | 1,5 | 0,3 |
| « O » | 85/15 | 34 | 3700 | 100 | 105 | 41 | 1,0 | 0,5 |

Les caractéristiques des coagglomérats de mannitol et d'amidon selon l'invention sont présentées dans le tableau 6 suivant.

**Tableau 6**

| Coagglomérats selon l'invention | Formes cristallines Alpha / Bêta (%) | Comprimabilité (N) | Note d'écoulement (s) | Granulométrie laser (D4,3 - µm) | Masse volumique tassée (g/ml) | Masse volumique aérée (g/ml) |
|---|---|---|---|---|---|---|
| « K » | 85/15 | 158 | 4 | 120 | 0,719 | 0,588 |
| « L » | 75/25 | 120 | 5 - 6 | 180 | 0,649 | 0,549 |
| « M » | 15/85 | 172 | 6 | 125 | 0,770 | 0,676 |
| « N » | 35/65 | 147 | 5 - 6 | 154 | 0,733 | 0,609 |
| « O » | 20/80 | 132 | 7 | 293 | 0,713 | 0,659 |

Les résultats obtenus démontrent que l'on obtient par granulation des coagglomérats conformes à l'invention équivalents à ceux obtenus par atomisation.

### Exemple 4 : Préparation de coagglomérats selon l'invention par atomisation du mélange d'une solution de mannitol et d'amidon granulaire introduit sous forme sèche dans la tour d'atomisation.

On prépare par atomisation selon l'invention trois coagglomérats consistant en mannitol et en amidon à un ratio de 80/20 avec du mannitol cristallisé et trois amidons granulaires différents, l'amidon de mais « Extra-blanc », la fécule de pomme de terre, et un amidon de mais waxy réticulé phosphate et stabilisé hydroxypropylé, commercialisé par la société demanderesse sous l'appellation CLEARAM® CR 20/10.

Les conditions opératoires de fabrication de ces coagglomérats sont figurées dans le tableau 7 suivant.

**Tableau 7**

| Coagglomérats Selon l'invention | Amidon mis en suspension dans la solution de mannitol | Matière sèche (%) | Pression (bars) | Buse (SPRAYING SYSTEM type SK) | Tp air amont (°C) | Tp du lit fluidisé statique (°C) | Tp air de sortie (°C) |
|---|---|---|---|---|---|---|---|
| « H » | Amidon de maïs extra blanc | 40 | 40 | 60*21 | 133 | 70 | 58 |
| « P » | Fécule de pomme de terre | 40 | 40 | 60*21 | 150 | 77 | 63 |
| « Q » | CLEARAM CR 20 10 | 40 | 40 | 60*21 | 150 | 77 | 63 |

Les caractéristiques des coagglomérats de mannitol et d'amidon selon l'invention sont présentées dans le tableau 8 suivant.

**Tableau 8**

| Coagglomérats selon l'invention | Formes cristallines Alpha / Bêta (%) | Comprimabilité (N) | Note d'écoulement (s) | Granulométrie laser (D4,3 - µm) | Masse volumique tassée (g/ml) | Masse volumique aérée (g/ml) |
|---|---|---|---|---|---|---|
| « H » | 85 | 312 | 5 - 6 | 173 | 0,568 | 0,483 |
| « P » | 90 | 227 | 4 - 5 | 165 | 0,654 | 0,556 |
| « Q » | 85 | 284 | 5 - 7 | 236 | 0,546 | 0,463 |

Le comportement des coagglomérats de mannitol selon l'invention est tout à fait satisfaisant en comprimabilité et en écoulement.

Le remplacement de l'amidon de mais extra blanc par tout autre type d'amidon granulaire est possible tout en préservant les caractéristiques essentielles du produit selon l'invention.

Il est donc possible de réaliser cet agglomérat quel que soit l'amidon granulaire à sa disposition, et donc de s'affranchir des problèmes de disponibilité, de culture régionale, mais aussi de tenir compte du souhait ou des contraintes des consommateurs (absence d'organisme génétiquement modifié) ou des patients (amidon de blé causant la maladie cœliaque).

Une modification chimique de l'amidon pour moduler ses propriétés est aussi réalisable dès lors que celui-ci reste granulaire.

### Exemple 5 : Remplissage de gélules sur une géluleuse automatique de type disque doseur, avec un coagglomérat selon l'invention et en comparaison, avec deux poudres de mannitol commercialisées par la société demanderesse

L'objet de ces essais est de déterminer le taux minimal de lubrifiant nécessaire à un fonctionnement correct de la géluleuse.

Pour cela, on utilise le stéarate de magnésium qui est le lubrifiant le plus utilisé en pharmacie.

Un mélange homogène de la poudre à tester et de stéarate de magnésium pharma végétal de BARLÖCHER (Allemagne) est préparé. Dans un pot d'une contenance de 1 litre, on place les quantités de produit à tester et de stéarate de magnésium de manière à atteindre une masse finale pour les deux de 300 g.

Les deux produits sont ensuite mélangés pendant 5 mn à l'aide d'un mélangeur épicycloïdale Turbula T2F de WILLY A. BACHOFEN (Suisse). Le mélange ainsi réalisé est dit lubrifié.

Le matériel utilisé pour remplir les dites gélules est une géluleuse In-Cap de DOTT. BONAPACE (Italie).

La taille des gélules choisie est le format « 2 » et toutes les pièces correspondant à ce format sont installées sur la géluleuse In-Cap.

Deux formats de tambour doseur sont possibles sur la géluleuse In-Cap. On utilise le grand modèle.

On règle les aiguilles de compression de la façon suivante : aiguille 1 = 27,5 mm, aiguille 2 = 29 mm, aiguille 3 = 32 mm et aiguille 4 = 35 mm.

La distance exprimée est celle mesurée entre la face supérieure de l'écrou de fixation de chaque aiguille et la face inférieure de l'écrou de réglage de chaque aiguille.

L'aiguille 1 est la première aiguille à entrer en action. L'aiguille 1 pénètre le plus profondément dans le disque doseur et l'aiguille 4 pénètre le moins profondément dans le disque doseur. Le disque doseur et le contre-disque ont une épaisseur de 14,5 mm.

Les enveloppes de gélules utilisées sont des Coni-Snap de CAPSUGEL (référence 2CS Natural Tr. Code 43.000).

On règle l'épaisseur du lit de poudre dans le tambour doseur afin d'obtenir des gélules remplies avec le mélange lubrifié et d'une masse finale de 310 mg.

L'essai est considéré comme satisfaisant si la production d'une série continue de 300 gélules est possible.

Aucune interruption d'aucune sorte n'est tolérée pendant la production de ces 300 gélules.

Les gélules ne sont pas refermées définitivement afin de pouvoir les ouvrir à nouveau et ainsi faire une observation des carottes qu'elles contiennent.

**Tableau 9**

| Produit testé | PEARLITOL® 200SD | PEARLITOL® 300DC | Agglomérat selon l'invention |
|---|---|---|---|
| Poids de la poudre incorporée dans la gélule (mg) | 310 | 370 | 280 |
| Taux minimal de lubrifiant (stéarate de magnésium) % | 3 | 3 | 1,5 |

Avec un taux de stéarate de magnésium inférieur à 3 % pour le PEARLITOL® 200SD et le PEARLITOL® 300DC (deux qualités de mannitol commercialisées par la société Demanderesse), ou inférieur à 1,5 % pour les coagglomérats selon l'invention, la géluleuse In-Cap ne peut pas fonctionner correctement.

Il se produit un grippage des carottes dans le disque doseur et l'aiguille d'éjection doit exercer une force anormalement élevée pour les pousser dans le corps des gélules.

Ce qui occasionne des problèmes qui vont d'une irrégularité de poids des gélules, à des arrêts fréquents de production, jusqu'à la casse de la géluleuse.

L'ajout d'une teneur élevée - 3 % - de stéarate de magnésium permet de résoudre le problème dans tous les cas, mais elle crée un retard à la dissolution de la gélule et donc de la biodisponibilité du principe actif, au détriment du soin apporté au patient.

En effet, le stéarate de magnésium est insoluble dans l'eau, et à cette concentration élevée de 3 %, il génère une couche barrière insoluble autour du contenu de la gélule, couche barrière insoluble qui ralentit fortement la dissolution de la gélule.

Les coagglomérats selon l'invention ne nécessitent qu'un faible taux de stéarate de magnésium ; la moitié du taux précédent. La couche barrière insoluble ne peut pas se créer ou est trop fragile pour limiter la dissolution et la biodisponibilité du principe actif.

### Exemple 6 : Remplissage de gélules avec des coagglomérats selon l'invention et un principe actif, le paracétamol.

Le matériel utilisé pour remplir les gélules est un simulateur monté sur une machine universelle de traction-compression INSTRON (USA). Le simulateur est un bloc de métal percé d'un trou vertical cylindrique fermé à la partie inférieure par une queue d'aronde mobile.

Le diamètre intérieur de ce trou est légèrement inférieur au diamètre intérieur d'une enveloppe de gélule de format « 0 ». La partie inférieure du bloc métallique est prévue pour recevoir un support maintenant la partie inférieure de l'enveloppe de la gélule dans l'axe du trou vertical.

Un cylindre métallique, appelé piston, d'un diamètre légèrement inférieur à celui du trou vertical est utilisé pour comprimer la poudre lubrifiée dans le bloc métallique et ainsi former la carotte.

Un mélange homogène du coagglomérat, du paracétamol (RHODAPAP® cristallisé fin de RHODIA) et de stéarate de magnésium pharma végétal de BARLÖCHER (Allemagne) est préparé.

Dans un pot d'une contenance de 1 litre, on place 147,75 g (49.25 %) de coagglomérat et 147,75g (49,25 %) de paracétamol. Les deux produits sont mélangés pendant 5 mn à l'aide d'un mélangeur épicycloïdale Turbula T2F de WILLY A. BACHOFEN (Suisse).

On ajoute 4,5 g (1,5 %) de stéarate de magnésium et on mélange à nouveau pendant 5 mn. Le mélange ainsi réalisé est dit lubrifié.

Les enveloppes de gélules de format « 0 » utilisées proviennent du Laboratoire LGA (Bandol France).

On introduit 500 mg de mélange lubrifié dans le simulateur. Le piston chargé de comprimer la poudre descend à une vitesse verticale constante de 20 mm par minute jusqu'à ce que la force appliquée sur le mélange lubrifié pour former la carotte soit de 600 N.

La carotte, une fois formée, et après retrait de la queue d'aronde mobile, est poussée par le piston dans la partie inférieure de l'enveloppe de la gélule.

Les gélules sont ensuite fermées manuellement.

Le temps de désagrégation des gélules ainsi préparées est mesurée selon la méthode préconisée par la Pharmacopée Européenne (P.E. 6.0, 01/2008 :20901, 2.9.1. Désagrégation des comprimés et des capsules), mesure réalisée sur 12 gélules.

**Tableau 10**

| | | | | | | |
|---|---|---|---|---|---|---|
| Coagglomérat selon l'invention | « A » | « H » | « I » | « J » | « P » | « Q » |
| Temps de délitement (s) | 151 | 147 | 175 | 161 | 164 | 159 |

Les gélules de paracétamol ainsi préparées présentent un temps de désintégration dans l'eau très court, inférieur à 3 minutes. Ce temps est très inférieur aux 15 minutes, temps de désagrégation imposé par la Pharmacopée Européennes pour les gélules.

Ces essais confirment que les coagglomérats selon l'invention permettent d'atteindre des temps de désintégration très courts.

### Exemple 7 : Réalisation de comprimés avec des coagglomérats selon l'invention et en comparaison avec une poudre de mannitol commercialisée par la société demanderesse

L'objet de ces essais est de déterminer le taux minimal de lubrifiant nécessaire à un fonctionnement correct de la presse à comprimer. Pour cela, on utilise le stéarate de magnésium qui est le lubrifiant le plus utilisé en pharmacie.

Un mélange homogène de la poudre à tester et de stéarate de magnésium pharma végétal de BARLÖCHER (Allemagne) est préparé.

Dans un pot d'une contenance de 1 litre, on place les quantités de produit à tester et de stéarate de magnésium de manière à atteindre une masse finale pour les deux de 300 g.

Les deux produits sont ensuite mélangés pendant 5 mn à l'aide d'un mélangeur épicycloïdal Turbula T2F de WILLY A. BACHOFEN (Suisse). Le mélange ainsi réalisé est dit lubrifié.

Les comprimés sont préparés à l'aide d'une presse alternative de laboratoire FROGERAIS AM commercialisé par la société SVIAC (France) équipée de poinçons concaves de diamètre 13 mm. Les comprimés à faces convexes et de diamètre 13 mm ont une épaisseur de 6 mm et un poids de 0,734 g.

Des teneurs décroissantes en stéarate de magnésium sont testées : 1,2 % ; 1,0 % ; 0,8 % ; 0,6 %, 0,4 % (% exprimé par rapport au mélange des deux poudres).

La présence de collage (une partie de la poudre reste collée à la surface du poinçon après éjection du comprimé) et de grippage (la tranche des comprimés présente des stries) est observée. La teneur minimale en lubrifiant correspond à la teneur à laquelle aucun de ces deux défauts n'est observé.

Pour le PEARLITOL® 200SD, poudre de mannitol commercialisée par la société demanderesse, cette teneur est de 1,2 %. Pour les coagglomérats selon l'invention, elle est de 0,6 %.

Une plus faible teneur en lubrifiant est favorable à la dissolution et à la biodisponibilité des actifs, de plus elle présage d'une plus grande facilité à produire industriellement les comprimés.

Une lubrification simple et aisée permet d'atteindre des cadences de production très élevées sans risque de déclassement ou arrêt suite à des problèmes de collage ou de grippage.

### Exemple 8 : Comparaison de coagglomérats selon l'invention et d'agglomérats de mannitol et d'amidon réalisés dans une tour d'atomisation simple effet

On prépare des agglomérats de mannitol cristallin et d'amidon granulaire, à des ratios mannitol/amidon respectivement de 80/20 et 50/50, dans une tour d'atomisation simple effet.

Pour cela, on utilise du mannitol cristallisé, commercialisé par la Société Demanderesse sous le nom PEARLITOL® 50C présentant un diamètre moyen volumique laser d'environ 50 µm et de l'amidon de maïs « extra-blanc ».

On prépare une solution de mannitol et d'amidon à la matière sèche désirée en dissolvant le mannitol cristallisé dans de l'eau déminéralisée à 55°C, et en y mettant en suspension l'amidon extra blanc de maïs.

L'agitation doit permettre d'obtenir une solution fluide, homogène et sans grumeaux. Les conditions opératoires de fabrication de ces agglomérats dans la tour d'atomisation simple effet commercialisée par la société NIRO (atomiseur Niro minor) sont présentées dans le tableau 11 suivant.

**Tableau 11**

| Agglomérats | Rapport mannitol / amidon | Matière sèche totale (%) | Tp air amont (°C) | Tp air de sortie (°C) |
|---|---|---|---|---|
| « R » | 80/20 | 32 | 200 | 63 |
| « S » | 50/50 | 32 | 200 | 63 |

Les caractéristiques des agglomérats de mannitol et d'amidon ainsi préparés dans une tour d'atomisation simple effet sont présentées dans le tableau 12 suivant.

**5 Tableau 12**

| Agglomérats | Ratio mannitol/amidon | Comprimabilité (N) | Note d'écoulement (s) | Granulométrie laser (D4,3 - µm) | Masse volumique tassée (g/ml) | Masse volumique aérée (g/ml) |
|---|---|---|---|---|---|---|
| « R » | 80/20 | 45 | infini | 37 | 0,649 | 0,877* |
| « S » | 50/50 | - | infini | 43 | 0,685 | 0,877* |

| | | | | | | |
|---|---|---|---|---|---|---|
| * L'écoulement dans l'entonnoir a été aidé grâce à une tige afin d'obtenir le volume aéré. | | | | | | |

Par rapport au comportement des coagglomérats selon l'invention, le comportement des agglomérats de mannitol et d'amidon réalisés dans une tour d'atomisation simple effet n'est absolument pas satisfaisant en termes de comprimabilité et d'écoulement.

La comprimabilité faible (agglomérat « R ») voire inexistante (agglomérat « S ») ne permet pas de formuler des comprimés qui conservent une dureté et donc une cohésion suffisante après incorporation d'une teneur élevée en principe actif.

De plus, les propriétés d'écoulement des agglomérats « R » et « S » sont si mauvaises qu'elles ne permettent pas de mesurer un temps d'écoulement (temps infini selon le test B).

## Revendications

1. Coagglomérats de mannitol cristallin et d'amidon granulaire **caractérisés en ce qu'**ils présentent:
- une comprimabilité, déterminée selon un test A, supérieure à 120 N, de préférence comprise entre 200 et 450 N
- une note d'écoulement, déterminée selon un test B, comprise entre 3 et 15 secondes, de préférence comprise entre 4 et 8 secondes et
- un ratio mannitol / amidon compris entre 99,5/0,5 et 50/50, et de préférence entre 95/5 et 70/30.

2. Coagglomérats selon la revendication 1, **caractérisés en ce qu'**ils présentent un diamètre moyen volumique laser D4,3 compris entre 60 et 500 µm, de préférence compris entre 100 et 250 µm.

3. Coagglomérats selon l'une quelconque des revendications 1 à à 2, **caractérisés en ce qu'**ils présentent
- une masse volumique aérée comprise entre 0,400 et 0,750 g/ml, de préférence comprise entre 0,450 et 0,650, et
- une masse volumique tassée comprise entre 0,500 et 0,850 g/ml, de préférence comprise entre 0,550 et 0,750 g/ml.

4. Coagglomérats selon l'une quelconque des revendications 1 à 3, **caractérisés en ce que** l'amidon est choisi dans le groupe constitué par l'amidon de maïs standard, l'amidon de maïs extra-blanc, la fécule de pomme de terre, pris seuls ou en combinaison.

5. Coagglomérats selon l'une quelconque des revendications 1 à 4, **caractérisés en ce que** le mannitol se présente à la fois sous les formes cristallines alpha et beta.

6. Procédé de préparation de coagglomérats de mannitol cristallin et d'amidon selon l'une quelconque des revendications 1 à 5, **caractérisé en ce qu'**il comprend les étapes suivantes :
a) préparer à une température comprise entre 45 et 65°C une solution de mannitol et d'amidon granulaire dans laquelle :
- le ratio mannitol/amidon est compris entre 99,5/0,5 et 50/50, et de préférence entre 95/5 et 70/30
- la matière sèche est comprise entre 25 et 45 % en poids sec,
b) maintenir ladite solution de mannitol et d'amidon à une température comprise entre 45 et 65°C,
c) atomiser ladite solution dans une tour d' atomisation de type MSD équipée d'une buse d'atomisation haute pression avec recyclage des fines particules en tête de tour,
d) récupérer les coagglomérats de mannitol et d'amidon ainsi obtenus.

7. Procédé de préparation de coagglomérats de mannitol cristallin et d'amidon selon l'une quelconque des revendications 1 à 5, **caractérisé en ce qu'**il comprend les étapes suivantes :
a) préparer à une température comprise entre 45 et 90°C une solution de mannitol dans laquelle la matière sèche est comprise entre 25 et 50 % en poids sec,
b) maintenir ladite solution de mannitol à une température comprise entre 45 et 90°C,
c) atomiser ladite solution dans une tour d' atomisation de type MSD équipée d'une buse d'atomisation haute pression avec recyclage des fines particules en tête de tour, en injectant via un doseur pondéral l'amidon sec dans le système de recyclage des fines dans un ratio mannitol/amidon compris entre 99,5/0,5 et 50/50, et de préférence entre 95/5 et 70/30
d) récupérer les coagglomérats de mannitol et d'amidon ainsi obtenus.

8. Procédé de préparation de coagglomérats de mannitol cristallin et d'amidon selon l'une quelconque des revendications 1 à 5, **caractérisé en ce qu'**il comprend les étapes suivantes :
a) préparer à une température comprise entre 45 et 65°C une solution de mannitol et d'amidon granulaire dans laquelle :
- le ratio mannitol/amidon est compris entre 99,5/0,5 et 50/50, et de préférence entre 95/5 et 70/30
- la matière sèche est comprise entre 25 et 45 % en poids sec,
b) maintenir ladite solution de mannitol et d'amidon à une température comprise entre 45 et 65°C,
c) granuler par pulvérisation de ladite solution dans un granulateur à lit d'air fluidisé,
d) récupérer les coagglomérats de mannitol et d'amidon ainsi obtenus.

9. Utilisation des coagglomérats des revendications 1 à 5 ou préparés selon le procédé de l'une quelconque des revendications 6 à 8 pour la fabrication de comprimés destinés aux domaines alimentaires, pharmaceutiques, phytosanitaires et détergents.

10. Comprimés composés de coagglomérats de mannitol et d'amidon granulaire des revendications 1 à 5 ou obtenus selon le procédé de l'une quelconque des revendications 6 à 8.

11. Utilisation des coagglomérats des revendications 1 à 5 ou préparés selon le procédé de l'une quelconque des revendications 6 à 8 pour la fabrication de gélules destinées aux domaines alimentaires et pharmaceutiques.

12. Gélules composées de coagglomérats de mannitol et d'amidon granulaire des revendications 1 à 5 ou obtenus selon le procédé de l'une quelconque des revendications 6 à 8.

## Patentansprüche

1. Koagglomerate aus kristallinem Mannitol und körniger Stärke, **dadurch gekennzeichnet, dass** sie folgendes aufweisen:
- eine gemäß einem Test A bestimmte Komprimierbarkeit von mehr als 120 N, bevorzugt zwischen 200 und 450 N,
- einen nach einem Test B ermittelten Fließwert zwischen 3 und 15 Sekunden, bevorzugt zwischen 4 und 8 Sekunden, und
- ein Mannitol/Stärke-Verhältnis zwischen 99,5/0,5 und 50/50 und bevorzugt zwischen 95/5 und 70/30.

2. Koagglomerate nach Anspruch 1, **dadurch gekennzeichnet, dass** sie einen mittleren Laservolumendurchmesser D4,3 zwischen 60 und 500 µm, bevorzugt zwischen 100 und 250 µm, aufweisen.

3. Koagglomerate nach einem der Ansprüche 1 bis 2, **dadurch gekennzeichnet, dass** sie aufweisen:
- eine lockere Rohdichte zwischen 0,400 und 0,750 g/ml, bevorzugt zwischen 0,450 und 0,650, und
- eine Klopfdichte zwischen 0,500 und 0,850 g/ml, bevorzugt zwischen 0,550 und 0,750 g/ml.

4. Koagglomerate nach einem der Ansprüche 1 bis 3, **dadurch gekennzeichnet, dass** die Stärke gewählt ist aus der Gruppe, umfassend Standard-Maisstärke, extra-weiße Maisstärke, Kartoffelstärke, allein oder in Kombination.

5. Koagglomerate nach einem der Ansprüche 1 bis 4, **dadurch gekennzeichnet, dass** Mannitol sowohl in alpha- als auch in beta-kristalliner Form vorliegt.

6. Verfahren zur Herstellung von Koagglomeraten aus kristallinem Mannitol und Stärke nach einem der Ansprüche 1 bis 5, **dadurch gekennzeichnet, dass** es die folgenden Schritte umfasst:
a) Herstellen einer Lösung aus Mannitol und körniger Stärke bei einer Temperatur zwischen 45 und 65°C, wobei:
- das Mannitol/Stärke-Verhältnis zwischen 99,5/0,5 und 50/50, und bevorzugt zwischen 95/5 und 70/30 liegt,
- die Trockenmasse zwischen 25 und 45 % des Trockengewichts beträgt,
b) Halten der Mannitol/Stärke-Lösung auf einer Temperatur zwischen 45 und 65 °C,
c) Zerstäuben der genannten Lösung in einem Zerstäubungsturm vom Typ MSD, der mit einer Hochdruckzerstäubungsdüse mit Rückführung der feinen Partikel am Kopf des Turms ausgestattet ist,
d) Rückgewinnen der so erhaltenen Mannitol- und Stärkekoagglomerate.

7. Verfahren zur Herstellung von kristallinen Mannitol- und Stärkekoagglomeraten nach einem der Ansprüche 1 bis 5, **dadurch gekennzeichnet, dass** es die folgenden Schritte umfasst:
a) Herstellen einer Mannitollösung, deren Trockenmasse zwischen 25 und 50 % Trockengewicht beträgt, bei einer Temperatur zwischen 45 und 90°C,
b) Halten der Mannitollösung auf einer Temperatur zwischen 45 und 90°C,
c) Zerstäuben der Lösung in einem Zerstäubungsturm vom Typ MSD, der mit einer Hochdruckzerstäubungsdüse mit Rückführung der feinen Partikel am Kopf des Turms ausgestattet ist, durch Einspritzen der Trockenstärke über einen Gewichtsdosierer in das Feinpartikel-Recycling-System in einem Mannitol/Stärke-Verhältnis zwischen 99,5/0,5 und 50/50, und bevorzugt zwischen 95/5 und 70/30,
d) Rückgewinnen der so erhaltenen Mannitol- und Stärkekoagglomerate.

8. Verfahren zur Herstellung von Koagglomeraten aus kristallinem Mannitol und Stärke nach einem der Ansprüche 1 bis 5, **dadurch gekennzeichnet, dass** es die folgenden Schritte umfasst:
a) Herstellen einer Lösung aus Mannitol und körniger Stärke bei einer Temperatur zwischen 45 und 65°C, in der:
- das Mannitol/Stärke-Verhältnis zwischen 99,5/0,5 und 50/50, und bevorzugt zwischen 95/5 und 70/30 liegt,
- die Trockenmasse zwischen 25 und 45 % Trockengewicht beträgt,
b) Halten der Lösung aus Mannitol und Stärke auf einer Temperatur zwischen 45 und 65°C,
c) Sprühgranulieren der Lösung in einem Wirbelschicht-Granulator,
d) Rückgewinnen der so erhaltenen Mannitol- und Stärkekoagglomerate.

9. Verwendung der Koagglomerate nach den Ansprüchen 1 bis 5 oder hergestellt nach dem Verfahren nach einem der Ansprüche 6 bis 8 zur Herstellung von Tabletten für den Lebensmittel-, Pharma-, Pflanzenschutz- und Waschmittelbereich.

10. Tabletten, bestehend aus Koagglomeraten aus Mannitol und körniger Stärke nach den Ansprüchen 1 bis 5 oder hergestellt nach dem Verfahren gemäß einem der Ansprüche 6 bis 8.

11. Verwendung der Koagglomerate nach den Ansprüchen 1 bis 5 oder hergestellt nach dem Verfahren nach einem der Ansprüche 6 bis 8 zur Herstellung von Kapseln für den Lebensmittel- und Pharmabereich.

12. Kapseln, bestehend aus Koagglomeraten aus Mannitol und körniger Stärke nach den Ansprüchen 1 bis 5 oder nach dem Verfahren gemäß einem der Ansprüche 6 bis 8.

## Claims

1. A coagglomerate of crystalline mannitol and of granular starch, wherein:
- the tableting capacity, determined according to test A, is greater than 120 N, preferably between 200 and 450 N,
- the flow grade, determined according to test B, is between 3 and 15 seconds, preferably between 4 and 8 seconds,
- the mannitol/starch ratio is between 99.5/0.5 and 50/50 and preferably between 95/5 and 70/30.

2. The coagglomerate as claimed in claim 1, wherein the laser volume mean diameter D4,3 is between 60 and 500 .µm, preferably of between 100 and 250 . µm.

3. The coagglomerate as claimed in claim 1 or 2, wherein: the aerated density is between 0.400 and 0.750 g/ml, preferably between 0.450 and 0.650 g/ml, and the tapped density is between 0.500 and 0.850 g/ml, preferably between 0.550 and 0.750 g/ml.

4. The coagglomerate as claimed in any one of claims 1 to 3, wherein the starch is chosen from the group consisting of standard corn starch, extra white corn starch and potato starch, taken alone or in combination.

5. The coagglomerate as claimed in in any one of claims 1 to 4, wherein the mannitol is provided both in the alpha and beta crystalline forms.

6. A process for the preparation of a coagglomerate of crystalline mannitol and of starch as claimed in anyone of claims 1 to 5, wherein it comprises the following steps:
a) preparing, at a temperature of between 45 and 65.degree. C., a solution of mannitol and granular starch in which: the mannitol/starch ratio is between 99.5/0.5 and 50/50 and preferably between 95/5 and 70/30, the solids content is between 25 and 45% as dry weight,
b) keeping said solution of mannitol and of starch at a temperature of between 45 and 65.degree. C.,
c) spray-drying said solution in an MSD-type spray-dryer equipped with a high pressure spray-drying nozzle with recycling of the fine particles at the spray-dryer top,
d) recovering the coagglomerates of mannitol and of starch thus obtained.

7. The process as claimed in in any one of claims 1 to 5, wherein it comprises the following steps:
a) preparing, at a temperature of between 45 and 90.degree. C., a mannitol solution in which the solids content is between 25 and 50% as dry weight,
b) keeping said mannitol solution at a temperature of between 45 and 90.degree. C.,
c) spray-drying said solution in an MSD-type spray-dryer equipped with a high pressure spray-drying nozzle with recycling of the fine particles in the spray-dryer top, the dry starch being injected via a weight metering device into the system for recycling the fine particles in a mannitol/starch ratio of between 99.5/0.5 and 50/50 and preferably between 95/5 and 70/30,
d) recovering the coagglomerates of mannitol and of starch thus obtained.

8. The process as claimed in in any one of claims 1 to 5, wherein it comprises the following steps:
a) preparing, at a temperature of between 45 and 65.degree. C., a solution of mannitol and of granular starch in which: the mannitol/starch ratio is between 99.5/0.5 and 50/50 and preferably between 95/5 and 70/30, the solids content is between 25 and 45% as dry weight,
b) keeping said solution of mannitol and starch at a temperature of between 45 and 65.degree. C.,
c) granulating said solution by spraying in a fluidized air bed granulator,
d) recovering the coagglomerates of mannitol and of starch thus obtained.

9. The use of the coagglomerate of claims 1 to 5 or prepared according to the process of any one of claims 6 to 8 in the manufacture of a tablet intended for the food, pharmaceutical, plant protection and detergent fields.

10. A tablet comprising the coagglomerate of mannitol and of granular starch of in anyone claims 1 to 5 or obtained by the process of any one of claims 6 to 8.

11. The use of the coagglomerate of claims 1 to 5 or prepared according to the process of any one of claims 6 to 8 in the manufacture of hard gelatin capsules intended for the food and pharmaceutical fields.

12. A hard gelatin capsule, composed of the coagglomerates of mannitol and of granular starch of claims 1 to 5 or obtained according to the process of any one of claims 6 to 8.
